# EUROPEAN PATENT APPLICATION

(11) **EP 1 602 329 A1**
(43) Date of publication of application: **07.12.2005**
(21) Application number: 05104729.8
(22) Date of filing: 01.06.2005
(51) Int. Cl.: A61B 5/15

(54) **Blood Collection Set With Venting Mechanism**

(30) Priority: 02.06.2004 US 576140 P
(71) Applicant: Becton, Dickinson and Company, Franklin Lakes, New Jersey 07417-1880 (US)
(72) Inventor: Nair, Hareesh, NJ 07086, Weehawken (US); Iskra, Michael, NJ 08807, Bridgewater (US); Ellis, Robert, NJ 07430 (US); Bennett, Michael, NJ 07901, Summit (US); Stewart, Marsden, NJ 07005 (US); Swenson, Kirk D., NJ 07006, North Caldwell (US); Conway, Hugh T., NJ 07044, Verona (US); Newby, C. Mark, NY 10987, Tuxedo (US); Bloch, Curtis, UT 84070, Sandy (US); Levy, Richard, SC 29150, Sumter (US); Wilkinson, Bradley, NJ 07508, North Haledon (US); Schneider, James C., NJ 07470, Wayne (US); Towns, Bryan, MD 21791, Union Bridge (US)
(74) Representative: Weber, Thomas

(57) **Abstract**

The blood collection set (10) includes an IV needle assembly (24), a length of flexible plastic tubing (46) extending from the IV needle assembly and a non-patient needle assembly (54). The set is formed with a venting mechanism that permits an outflow of air, while blocking an outflow of blood or other fluids. Thus, the venting mechanism enables air that had existed in interior portions of the blood collection set to be vented allowing for greater flash visualization on venous entry, and avoids the need to employ a discard tube.

## Description

### BACKGROUND OF THE INVENTION

This application claims priority to U.S Provisional Patent Application Serial No 60/576,140, which was filed on June 1, 2004.

### 1. Field of the Invention

The subject invention relates to a blood collection set with self-venting features

### 2. Description of the Related Art

Phlebotomy procedures often are carried out using a blood collection set. A typical blood collection set includes an IV needle assembly with an IV cannula that has a proximal end, a sharply pointed distal end and a lumen extending between the ends. The needle assembly also includes a plastic IV hub with a proximal end, a distal end, and a passage extending between the ends. The proximal end of the IV cannula is mounted in the passage of the IV hub so that the lumen through the IV cannula communicates with the passage through the IV hub. The needle assembly may further include a shield for shielding the IV cannula after use and a packaging cover for safely covering the IV cannula prior to use. Packaging covers typically are rigid tubes with a proximal end that can be telescoped over the TV cannula and frictionally engaged with the distal end of the IV hub. Shields for blood collection sets have taken many forms. Some shields are telescoped over the IV hub and can be moved from a proximal position where the cannula is exposed to a distal position where the cannula is shielded. Other shields are hinged to the IV hub and can be rotated from an open position where the IV cannula is exposed to a closed position where the IV cannula is shielded. A needle assembly for a blood collection set also may include two flexible wings that project transversely from the IV hub or from the shield. The wings can be folded into face-to-face relationship with one another to effectively define a handle that facilitates manipulation of the needle assembly. The wings then can be rotated away from one another and held against the skin of the patient.

Blood collection sets also include a length of flexible plastic tubing. The tubing has a distal end that is connected to the proximal end of the IV hub. The tubing also has a proximal end that is connected to a plastic fitting. Thus, fluid communication is provided between the lumen of the IV cannula and the plastic fitting at the proximal end of the flexible tubing. The plastic fitting may be a female luer fitting that can be connected to a male luer fitting. The fitting then can be placed in communication with a reservoir or container for collecting a sample of blood.

Phlebotomy procedures often employ evacuated tubes, such as the VACUTAINER® brand of evacuated tubes sold by Becton Dickinson and Company. Evacuated tubes often are used with a tube holder that has a proximal end, a distal end, and a tubular side wall extending between the ends. The proximal end of the holder is widely open and is configured for slidably receiving the evacuated tube. The distal end of the holder typically includes an end wall with a mounting aperture. The mounting aperture includes internal threads or other mounting structures.

The tube holder may be used with a non-patient needle assembly that has a non-patient hub with external surface configurations for mounting in the mounting aperture of the holder. The non-patient needle assembly further includes a non-patient cannula extending proximally from the hub and a multiple sample sleeve telescoped over the non-patient cannula and mounted to the proximal end of the hub. The hub of the non-patient needle assembly can be threaded or otherwise engaged in the mounting aperture of the tube holder so that the non-patient needle and the multiple sample sleeve project into the tube receiving chamber of the holder.

The blood collection set may be used by mounting the fitting at the proximal end of the flexible plastic tubing to the distal end of the hub of the non-patient needle assembly. The packaging shield that covers the non-patient cannula then may be removed, and the hub of the non-patient needle assembly may be engaged with the tube holder. The medical practitioner then grips the IV needle assembly and removes the packaging cover from the IV cannula. The gripping of the TV needle assembly may include folding the flexible wings into face-to-face engagement and gripping the folded wings between a thumb and forefinger, the pointed distal end of the IV cannula then is urged into a targeted blood vessel. The wings then may be folded into engagement with the skin of the patient and may be taped in position An evacuated tube then is urged into the open proximal end of the blood collection tube holder so that the non-patient needle pierces the stopper of the evacuated tube. As a result, the blood vessel of the patient is placed in communication with the interior of the evacuated tube, and the pressure differential between the blood vessel and the evacuated tube will generate a flow of blood through the IV cannula, through the passage of the IV hub, through the flexible tubing, through the non-patient hub and finally through the non-patient needle and into the evacuated tube.

It will be appreciated that a significant volume of an must be displaced before blood enters the evacuated tube. This air will be displaced by the flowing blood and will be urged into the evacuated tube. The flow of air into the evacuated tube increases the air pressure in the tube and offsets the pressure differential that generates the flow of blood from the patient to the evacuated tube. Thus, blood flow is slowed Blood flow into the blood collection tube may stop when the pressure in the tube equals the fluid pressure of the blood. In effect air from the blood collection set reduces the volume of blood collected into the tube. The reduced blood volume can be undesirable such as when it adversely affects the ratio of additive to blood within the tube. An example is when the tube contains the additive citrate for clotting time studies in which the ratio of blood to citrate is critical.

Medical practitioners have several approaches for addressing problems relating to air in a blood collection set at the start of a phlebotomy procedure. For example, the first tube of collected blood may be considered a discard tube. Thus, the evacuated tube will remain in communication with the non-patient needle until blood begins to flow into the tube. The tube then will be removed and discarded and a second tube will be inserted into the holder for collecting a sample that can be used reliably. This approach adds to the cost and time of the procedure and wastes blood. Some medical practitioners try to vent air from the system before the first blood collection tube is placed in communication with the non-patient needle. This approach also wastes blood and can lead to contamination or accidental sticks depending upon the method of venting

The typical needle hub is formed from an opaque plastic material, and plastic tubing often is formed from a translucent plastic material. Neither the opaque plastic material not the translucent flexible tubing provide a clear indication of venous or arterial access. Blood flow into an evacuated tube does provide an indication of venous or arterial access. However, the initial movement of air into the evacuated tube is delayed until the evacuated tube is added onto the non patient needle. Thus, a medical practitioner may have a delayed indication of venous or arterial access and may incorrectly assume that the blood vessel was not accessed properly. In these situations, the medical practitioner may try to access the blood vessel again even though the initial access was successful. Accordingly, the patient may be subjected to unnecessary trauma during a repeated attempt to access the targeted blood vessel. Thus, improved techniques for dealing with the issue of air trapped in tubing would be desirable.

### SUMMARY OF THE INVENTION

The invention is a self-venting blood collection set with a self-venting mechanism that permits escape of air during use, and which, typically, also prevents an outflow of fluid, such as blood. As used herein, venting mechanism indicates one or more features or elements that provide venting of air, but which, typically, prevent fluid from passing through. Thus, air under venous pressure will be allowed to escape from the blood collection set through the mechanism until blood reaches the venting mechanism the venting mechanism then will seal, or prevent blood flow through or around it, to prevent blood leakage and allow blood to be collected into evacuated collection tubes or into other appropriate blood collection receptacles. The invention thus provides good flash visualization, as well as the capability to provide a blood collection set that docs not requite a discard tube, without affecting accepted blood collection processes A variety of venting mechanisms, venting media and venting locations are suitable, as set forth below (As used herein, venting mechanism indicates the combination of elements, configurations, materials, etc, that provide the venting. As used herein, venting media indicates the actual clement that vents the air, e g., plug, coating, finish, etc.)

the blood collection set preferably includes an IV needle assembly, a length of flexible plastic tubing extending from the IV needle assembly and a non-patient needle assembly. The venting mechanism preferably is disposed on or neat the non-patient needle assembly to permit venting of a maximum amount of the air that is in the blood collection set prior to the initiation evacuated tube use.

The IV needle assembly typically comprises an IV hub having a proximal end, a distal end and a passage extending between the ends. The IV needle assembly typically comprises an IV cannula having a proximal end mounted in the passage of the TV hub, a pointed distal end projecting distany from the IV hub and a lumen that communicates with the passage through the IV hub. The flexible tubing is connected to the proximal end of the IV hub. The IV needle assembly typically includes a packaging cover that protectively encloses the IV needle cannula prior to use the packaging cover is removed immediately prior to use to permit access to the IV cannula. The IV needle assembly may further include a protective shield that is moveable relative to the IV cannula from an open position where the IV cannula is exposed to a closed position where the IV cannula is substantial1y shielded The shield protects against accidental sticks with the used IV cannula. A pair of flexible wings may be mounted to the IV hub or to the shield to facilitate manipulation of the IV needle assembly.

The non-patient needle assembly includes a non-patient hub having a proximal end and a distal end The non-patient needle assembly further includes a non-patient cannula having a distal end securely mounted in the hub, a proximal end projecting proximally from the non-patient hub and a lumen that communicates with the passage through the non-patient hub. A multiple sample sleeve is typically mounted over the non-patient cannula and seemed to the proximal end of the non-patient hub External portions of the non-patient hub near the proximal end thereof may be formed with an array of external threads or other mounting structure to enable the non-patient needle assembly to be mounted to a collection tube holder or other such medical device. Or, the holder may be pre-attached with the non-patient needle assembly The blood collection set may further include a fitting mounted to the proximal end of the flexible plastic tubing and configured for mating with the distal end of the non-patient hub. For example, the fitting may be a female luer fitting that can be engaged with the male luer taper at the distal end of the non-patient hub

In one embodiment, the venting mechanism is located at or near the -non-patient hub, e.g., in the hub itself near the distal end of the non-patient needle or in the tubing itself at a proximal end thereof. The venting mechanism thereby provides communication between the passage and the surrounding environment either through the passage itself or through the non-patient hub Alternatively, wherein the venting mechanism is located in the tubing, any location along the tubing is possible.

In a further embodiment, the venting mechanism location is in a space between a female and male luer interface or within a female luer and therefore will be at or near to the non-patient needle so that only a small amount of air will be collected with the first sample of blood.

In another embodiment, the venting mechanism is located beyond the non-patient cannula proximal end, which means that the air passes through the non-patient cannula proximal end from which blood is drawn, and then through the vent Specifically, air is vented from the fluid passage and out of the non-patient cannula proximal end where it further flows through the space between needle exterior and multiple sample sleeve. The air then flows through the venting mechanism, which may be at the non-patient barb, the non-patient hub thread, the non-patient hub body, the multiple sample sleeve, or other location or combination of locations that are beyond the non-patient cannula proximal end. The collection tube, which is applied at the non-patient cannula proximal end, draws blood from only the fluid passage and not from the vent space. This embodiment thus enables blood to flow through the entire collection path for full tubing flash, eliminating the need for a discard tube and maintaining the desired blood to additive ratio. It also avoids the blood specimen coming in contact with the vent, which could potentially cause platelet activation, contamination or other undesirable result. It also avoids air being sucked back into the fluid passage when the evacuated tube is applied

In a further embodiment, the venting mechanism is an opening in the side of the non-patient cannula, combined with a vent media, which vents air but not blood, e.g. a vent plug consisting of a hydrophobic material.

Another embodiment of this invention has a venting mechanism comprised of a unified non-patient hub that is at least partially constructed of porous material such as sintered plastic, ceramic on metal. The porous material can be an anged to provide venting of air either before that air enters the non-patient cannula, or after the air flows through the cannula, out the proximal end, and into the space between the cannula and a multiple sample sleeve. The porous material provides venting of the air but blocks leakage of the blood. In the typical embodiment, the porous material is hydrophobic. The porous material may further contain or be coated with materials that swell upon wetting to further contain the blood Other venting methods are also possible. The internal passage wall's surface may be coated with a scalant to prevent contamination of the blood sample by the porous material. This embodiment enables blood to flow through the entire collection path for full tubing flash, and also enables elimination of the waste tube or variability in blood to additive ratio. Optionally, the hub can be permanently bonded to a tube holder obviating the need or inconvenience of threaded connections. Bonding to the holder may be accomplished by solvent, welding, heat, pressure or and other convenient means or combination thereof. Such an integrated device is highly efficient to manufacture, and promotes safe medical practice by having the holder be discarded with the needle.

In a further embodiment, the venting mechanism involves venting air through a side opening located somewhere along the fluid passage to the exterior, where the opening is covered by a venting material having a shape which mechanically holds the venting material in or on the opening. Preferably, the vent material is hydrophobic such that the surface tension also prevents leakage, the vent media material in this embodiment typically has an elastic property and shape such that spring energy holds the vent material onto the device For example, it is possible to use a C-shaped vent in which distortion of the shape is requited for the vent to stretch over the receiving structure on the hub. Once the vent is placed over the receiving structure, it is released and fully maintained in place using it's own resiliency and in absence of bonding materials such as epoxies, which could be disadvantageously absorbed into the vent. The vent mechanism of this embodiment could alternatively involve first compressing a vent material, placing the material into the opening, and releasing the vent material to expand into the opening This embodiment enables efficient mass production.

In a further embodiment, the venting mechanism utilizes a branch in the fluid passage, e.g., a "Y" or "T". The branching may be at any location or locations along the fluid passage, but is preferably at the proximal end such as at the non-patient hub. The branching may be in the form of a separate component added into the fluid passage such as in between the female and male luer fittings or it may be integral within the hub. The branching includes some type of vent media, as discussed herein.

### BRIEF DESCRIPTION OF THE DRAWINGS

FIG. 1A is a perspective view of a blood collection set and collection tube holder.

FIG. 1B is a top plan view of the blood collection set and collection tube holder shown in FIG. 1.

FIG. 2 is side elevation view of the non-patient needle assembly, partly in section.

FIG. 3 is a cross-sectional side view of an embodiment of the female luer in the non-patient needle assembly of the blood collection set.

FIG. 4 is also a cross-sectional side view similar to FIG. 3, but showing an alternate embodiment of the invention

FIG. 5 is a side elevation view of FIG. 4 from the aspect of 7-7

FIG. 6 is also a cross-sectional side view similar to FIG. 3, but showing an alternate embodiment, of the invention,

FIG. 7 is a side elevation view of FIG. 6

FIG. 8 is a cross-sectional side view of the female to male luer interface in this embodiment of the non-patient needle assembly of the blood collection set.

FIG. 9 is a perspective view of an embodiment of a female luer design in the non-patient needle assembly of the blood collection set.

FIG. 10 is a perspective view similar to FIG. 9, but showing an alternate embodiment of the invention

FIG. 11 is a perspective view similar to FIG. 9, but showing an alternate embodiment of the invention.

FIG. 12 is a perspective view similar to FIG. 9, but showing an alternate embodiment of the invention

FIG. 13 is a cross-sectional side view of the female to male luer interface in this embodiment of the non-patient needle assembly of the blood collection set.

FIG. 14 is a cross-sectional side view of an embodiment of the male luer non-patient assembly

FIG. 15A is also a cooss-scctional side view similar to FIG. 14, but showing an alternate embodiment of the invention.

FIG. 15B is also a cross-sectional side view similar to FIG. 14, but showing an alternate embodiment ot the invention

FIG. 15C is a magnified view of FIG. 15R from the aspect of Detail B.

FIG. 16A is also a cross-sectional side view similar to FIG. 14, but showing an alternate embodiment of the invention

FIG. 16B is a side elevation view of FIG. 16A from the aspect of X-X

FIG. 17A is also a cross-sectional side view similar to FIG. 14, but showing an alternate embodiment of the invention.

FIG. 17B is a side elevation view of FIG. 17A from the aspect of Y-Y.

FIG. 18 is also a cross-sectional side view similar to FIG. 14, but showing an alternate embodiment of the invention.

FIG. 19 is also a cross-sectional side view similar to FIG. 14, but showing an alternate embodiment of the invention

FIG. 20 is also a cross-sectional side view similar to FIG. 14, but, showing an alternate embodiment of the invention.

FIG. 21 is a cross-sectional side view of an embodiment of the interface between the non-patient cannula hub the mate luer hub

FIG. 22 is a cross-sectional side view of an embodiment of the non-patient needle assembly.

FIG. 23 is a cross-sectional side view of an embodiment of the non-patient needle assembly of the blood collection set.

FIG. 24 is also a cross-sectional side view similar to FIG. 23, but showing an alternate embodiment of the invention,

FIG. 25A is a cross-sectional side view of an embodiment of the non-patient needle assembly of the blood collection set.

FIG. 25B is a side elevation view of FIG. 25A from the aspect of R-R.

FIG. 26 is a perspective view of an embodiment of the female and male luer interface in the non-patient needle assembly of the blood collection set.

FIG. 27 is a perspective view of an embodiment of the non-patient barb multiple sample sleeve interface in the non-patient needle assembly of the blood collection set.

FIG. 28 is a cross-sectional view of an embodiment of the breathable cord design.

FIG. 29 is also a cross-sectional side view similar to FIG. 28, but showing an alternate embodiment of the invention

FIG. 30 is also a cross-sectional side view similar to FIG. 14, but showing an alternate embodiment of the invention.

FIG. 31 is also a cross-sectional side view similar to FIG. 14, but showing an alternate embodiment of the invention

FIG 32 is also a cross-sectional side view similar to FIG. 14, but showing an alternate embodiment of the invention

FIG. 33 is also a cross-scctional side view similar to FIG. 14, but showing an alternate embodiment of the invention

FIG. 35 is also a cross-sectional side view similar to FIG. 14, but showing an alternate embodiment of the invention

FIG. 36 is also a cross-sectional side view similar to FIG. 14, but showing an alternate embodiment of the invention.

FIG. 37 is also a cross-sectional side view similar to FIG. 14, but showing an alternate embodiment of the invention

FIG. 38 is a cross-sectional side view of an embodiment of the non-patient needle assembly of the blood collection set.

FIG. 39 is a cross-sectional side view of the flexible tubing in an embodiment of the blood collection set.

FIG. 40 is a perspective view of one embodiment of the blood collection set

FIG. 41 is a perspective view simi!ar to FIG. 40, but showing an alternate embodiment of the invention

FIG. 42 is a cross-sectional side view of the non-patient needle assembly of the blood collection set.

### DETAILED DESCRIPTION

The invention is a self-venting blood collection set with a self-venting mechanism that permits escape of air during use which, typically, also prevents an outflow of fluid, such as blood. As used herein, venting mechanism indicates one or more features or elements that provide venting of air, but which, typically, prevent fluid from passing through.

It should be noted that the vent media could be, for example, a distinct physical element such as a plug of insert, a integral portion of a device that has been treated such as by laser drilling or has been formed in whole or in part from a porous material, or a coating, layer, etc. formed by disposing a material onto the device, e.g., by dipping, coating, spraying or the like.

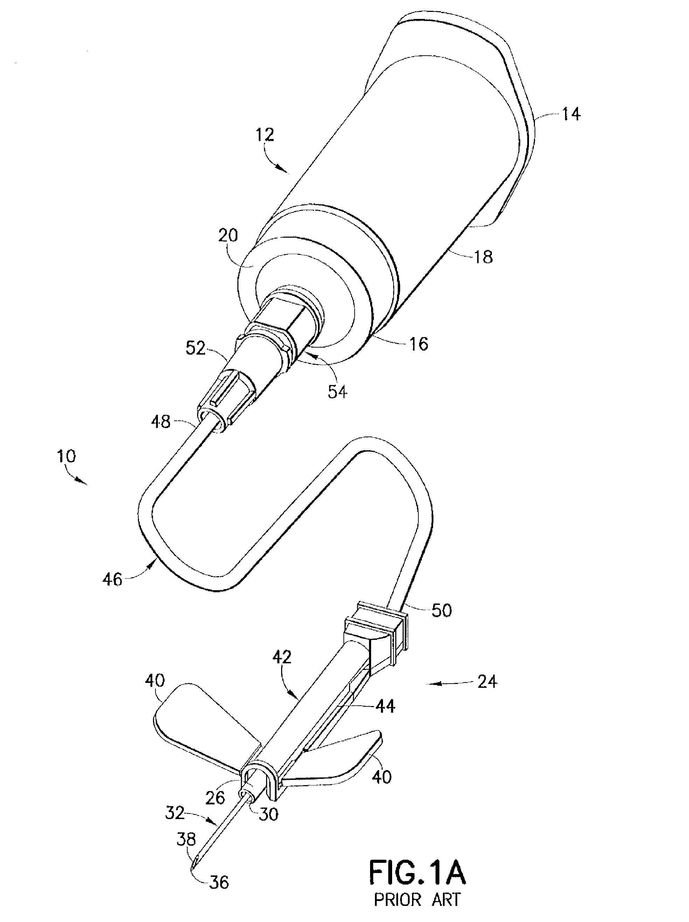

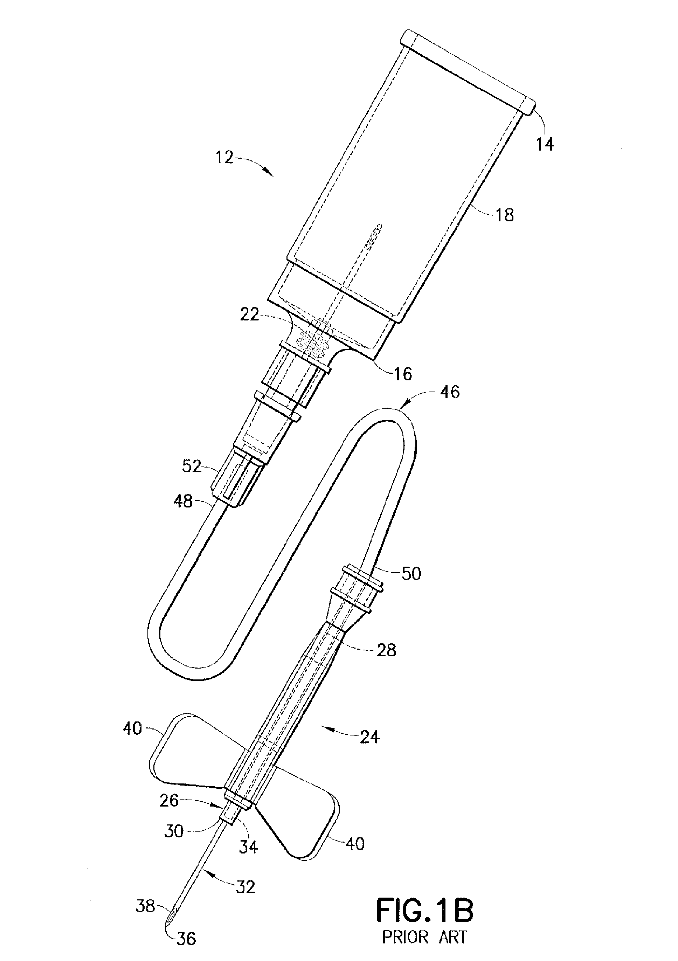

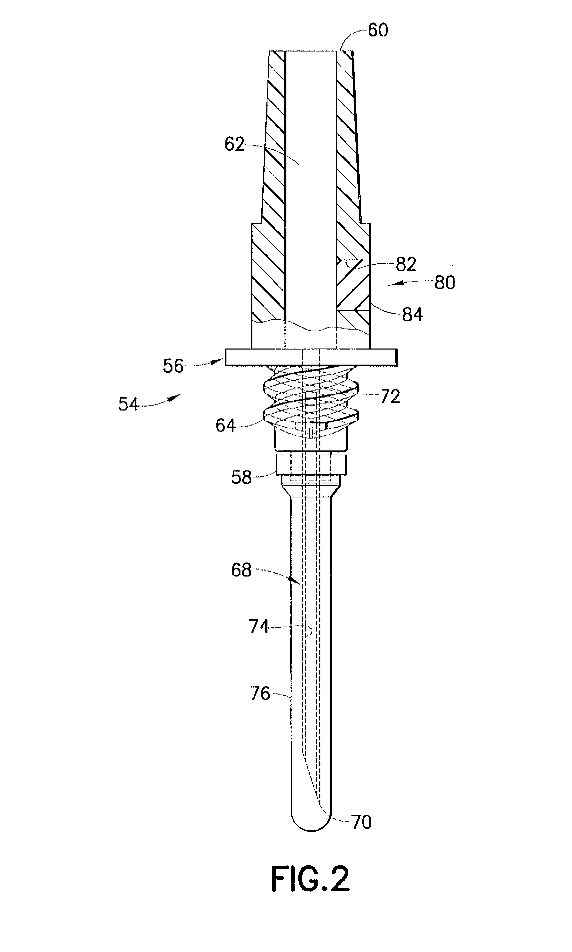

A prior art blood collection set in accordance with the subject invention is identified generally by the numeral **10** in FIGS. 1A and 1B. Blood collection set **10** is employed in this embodiment with a collection tube holder **12.** Holder **12** has a proximal end **14,** a distal end **16** and a tubular sidewall **18** extending between the ends. Proximal end **14** of holder **12** is widely open and defines an entry to a tube receptacle within sidewall **18.** Thus, an evacuated collection tube can be slid in a proximal-to-distal direction through open proximal end **14** of holder **12** toward distal end **16.** Distal end **16** of holder **12** is characterized by an end wall **20.** End wall **20** is formed with an internally threaded mounting aperture **22,** as shown in FIG. 2

Blood collection set **10** includes an IV needle assembly **24** that comprises an IV hub **26**. IV hub **26** includes a proximal end **28,** a distal end **30** and a passage (not shown) extending between the ends IV needle assembly **24** further includes an IV cannula **32** with a proximal end **34**, a pointed distal end **36** and a lumen **38** extending between the ends. Proximal end **34** of IV cannula **32** is mounted securely in the passage of IV hub **26**. Thus, lumen **38** through IV cannula **32** communicates with the passage through IV hub **26**. Flexible wings **40** are mounted to IV hub **26** at a location near distal end **30**. Wings **40** can be folded into face-to-face relationship with one another for convenient gripping between a thumb and forefinger to enable manipulation of IV needle assembly **24** Wings **40,** however, also can be rotated into a substantially coplanar disposition for taping to the skin of a patient.

IV needle assembly **24** further includes a tubular shield **42** that is telescoped over IV hub **26**. Shield **42** is formed with transverse slots **44** that slidably receive wings **40.** Thus, shield **42** can be slid from a proximal position, as shown in FIGS. 1A and 1B to a distal position IV cannula **32** is exposed for use when shield **42** is in the proximal position shown in FIGS. 1A and 1B. However, IV cannula **32** is substantially surrounded by shield **42** when shield **42** is moved to the distal position. Additionally, slots **44** in shield **42** are configured to lockingly engage wings **40** when shield **42** is in the distal position to prevent or complicate a re-exposure of IV cannula **32**. The shield illustratcd in FIGS. 1A and 1B is one of many optional shield designs that can be incorporated into blood collection set **10**. Other designs may provide wings mounted directly to the shield. Still other designs may provide a hinged shield mounted to IV hub **26**. In still other designs, a shield may be entirely separate from IV needle assembly **24** or a shield may not be provided at all Moreover, an unshielded set is also possible according to the invention.

Blood collection set **10** further includes a length of flexible plastic tubing **46.** Tubing **46** includes opposite proximal and distal ends **48** and **50** and a passage extending between the ends. Distal end **50** of tubing **46** is securely mounted to proximal end **28** of IV hub **26** so that the passage through IV hub **26** communicates with the passage through tubing **46**. A female luer fitting **52** is securely mounted to proximal end **48** of tubing **46.**

Blood collection set **10** further includes a non-patient needle assembly **54,** as shown in FIG. 2. Non-patient needle assembly **54** includes a non-patient hub **56** with a proximal end **58,** a distal end **60** and a fluid passage **62** extending between the ends exterior surface regions of non-patient hub **56** substantially adjacent proximal end **58** define an array or external threads **64** configured for threaded engagement with the internal threads formed in mounting aperture **22** of collection tube holder **12**. External surface regions of non-patient hub **56** adjacent distal end define a male luer taper **66** configured for mating with female luer fitting **52.** Non-patient needle assembly **54** further includes a non-patient cannula **68** having a pointed proximal end **70**, a distal end **72** and a lumen **74** extending between the ends. Distal end **72** of non-patient cannula **68** is mounted securely in passage **62** through non-patient hub **56** and aligns substantially with external threads **64** on non-patient hub **56.** Non-patient needle assembly **54** further includes a multiple sample sleeve **76** mounted over non-patient cannula **68** and securely engaged with proximal end **58** of non-patient hub **56.** Multiple sample sleeve **76** effectively functions as a valve that prevents a flow of fluid from non-patient cannula **68.** However, multiple sample sleeve **76** can be pierced by pointed proximal end **70** of non-patient cannula **68** in response to forces generated by a stopper on an evacuated collection tube.

Blood collection set **10** is employed by folding wings **40** into face-to-face engagement with one another and gripping wings **40** between a thumb and forefinger. Any packaging cover that may be mounted over IV cannula **32** then is removed and discarded. Pointed distal end **36** of IV cannula **32** then is urged into a targeted blood vessel. The healtheare practitioner then may release the grip on wings **40**, and if long term access to the blood vessel is required, wings **40** may be taped into face-to-face engagement with the skin of the patient. Blood collection set **10** includes a plurality of internal spaces that will initially be at ambient air pressure. These internal spaces include lumen **38** through IV cannula **32,** the passage through IV hub **26,** the passage through flexible tubing **46,** passage **62** through non-patient hub **56** and lumen **74** through non-patient cannula **68.** The venous or arterial access achieved with IV cannula **32** places these interior spaces of blood collection set **10** in communication with the pressure of the blood in the patient. Blood pressure exceeds the ambient air pressure. Accordingly, the pressure of air in the above-referenced internal spaces will increase, and blood will begin to flow into these internal spaces As discussed above, prior art systems may reach equilibrium as the air pressure within the blood collection set increases in response to a reduction of volume caused by the inflow of blood. Hence, a portion of the internal spaces in the prior art system may remain filled with air at a pressure substantially equal to the venous or arterial pressure. Stated differently, a prior art system will include its original volume of air in the space between the proximal end of the non-patient needle and the blood that enters the blood collection set. This high-pressure air will escape into the first evacuated collection tube that is placed in communication with the non-patient needle. Hence, the first collection tube employed with prior art systems normally is a discard tube. With the subject invention, however, the communication of blood at venous or arterial pressure with the interior spaces of blood collection set **10** will urge air through a venting mechanism. Various embodiments of such venting mechanisms arc described in detail below

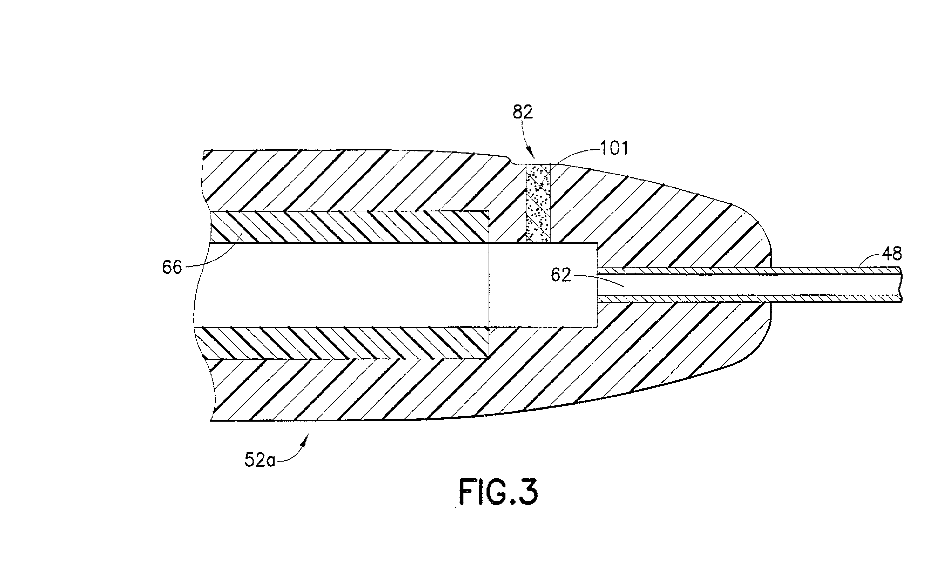

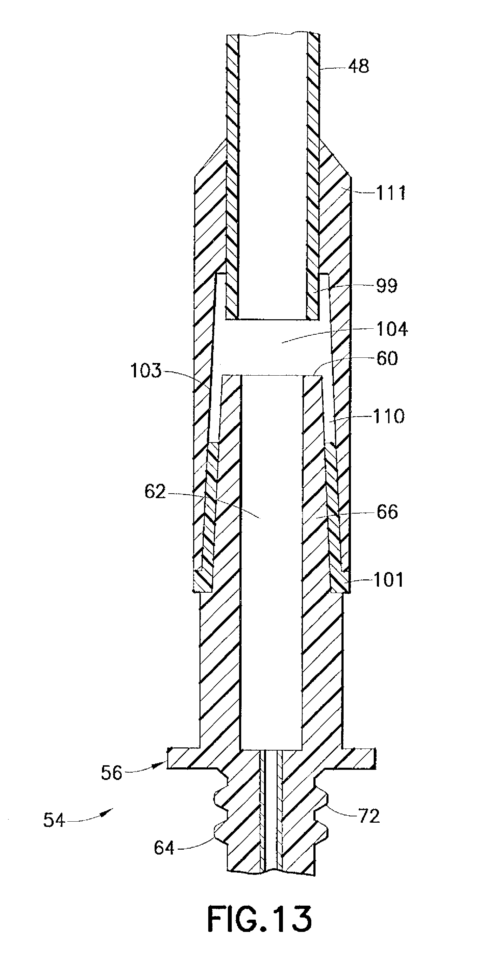

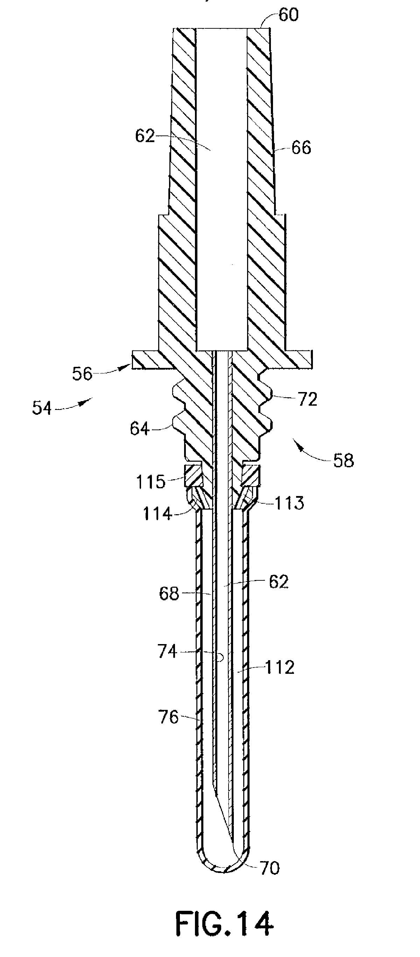

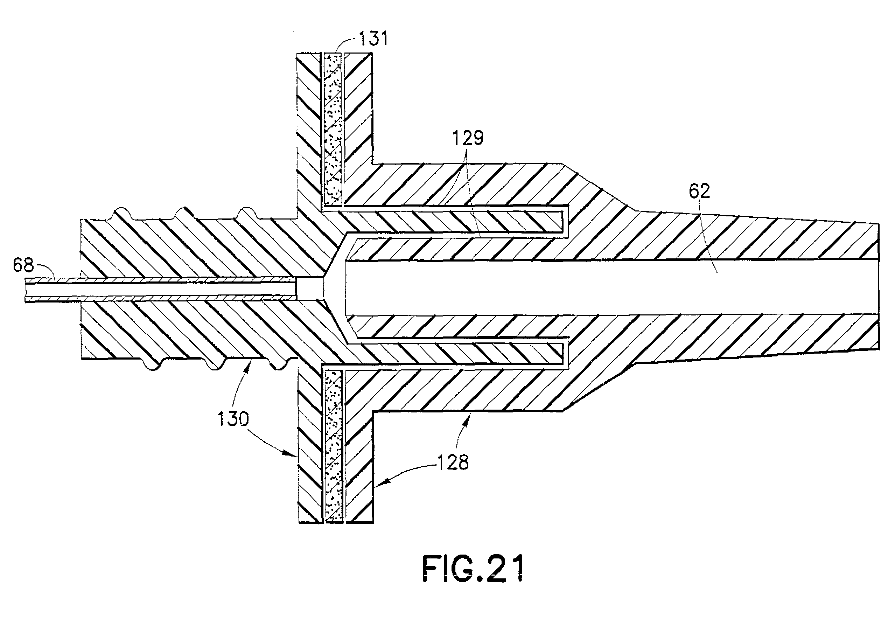

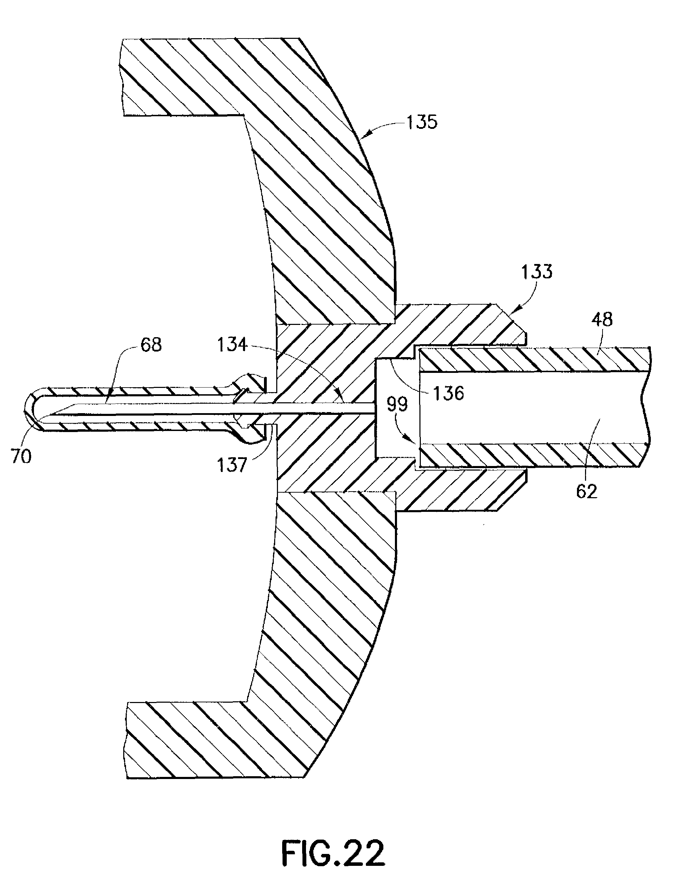

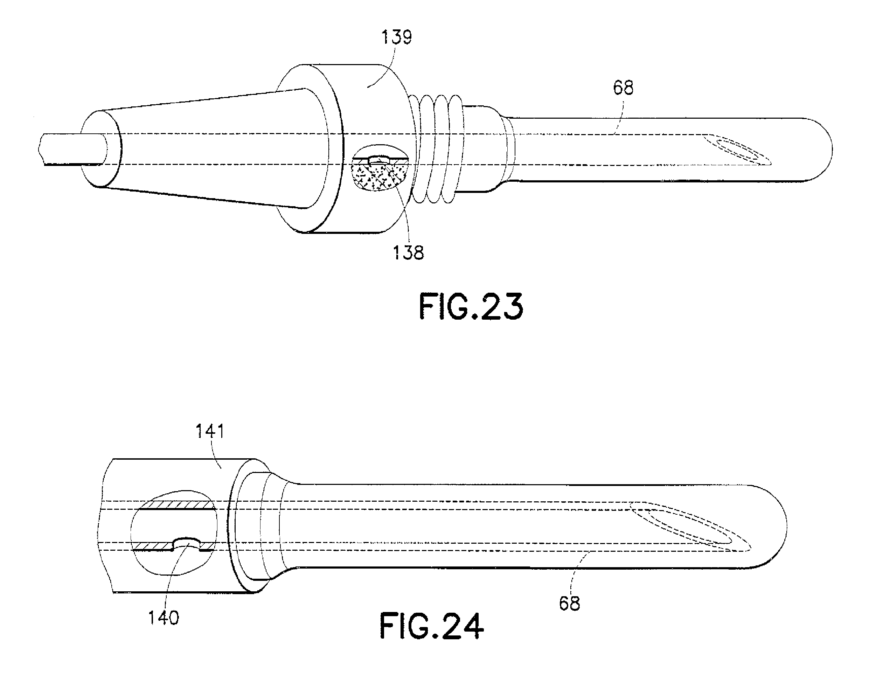

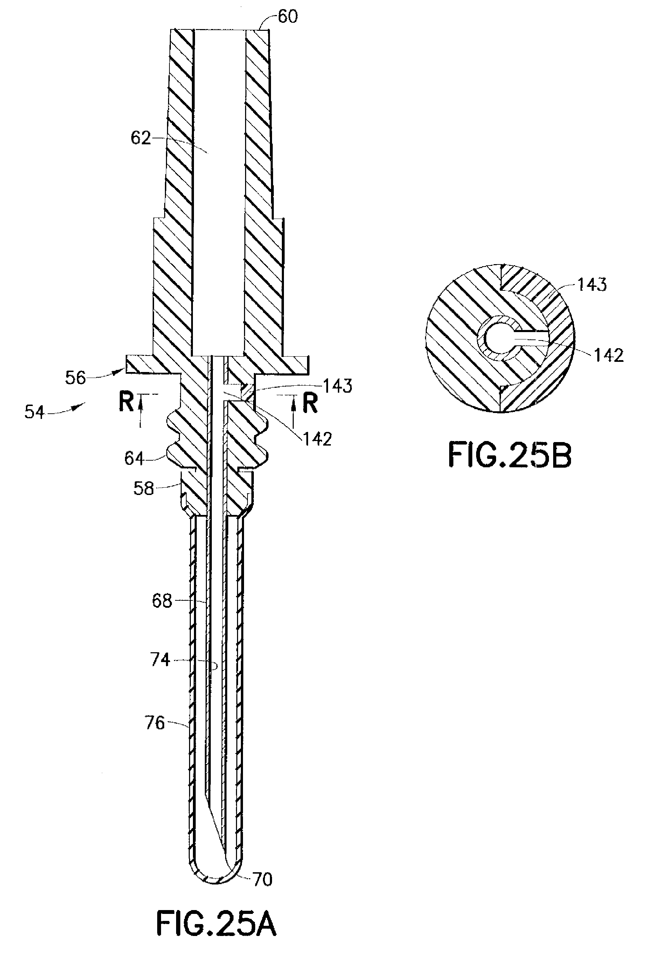

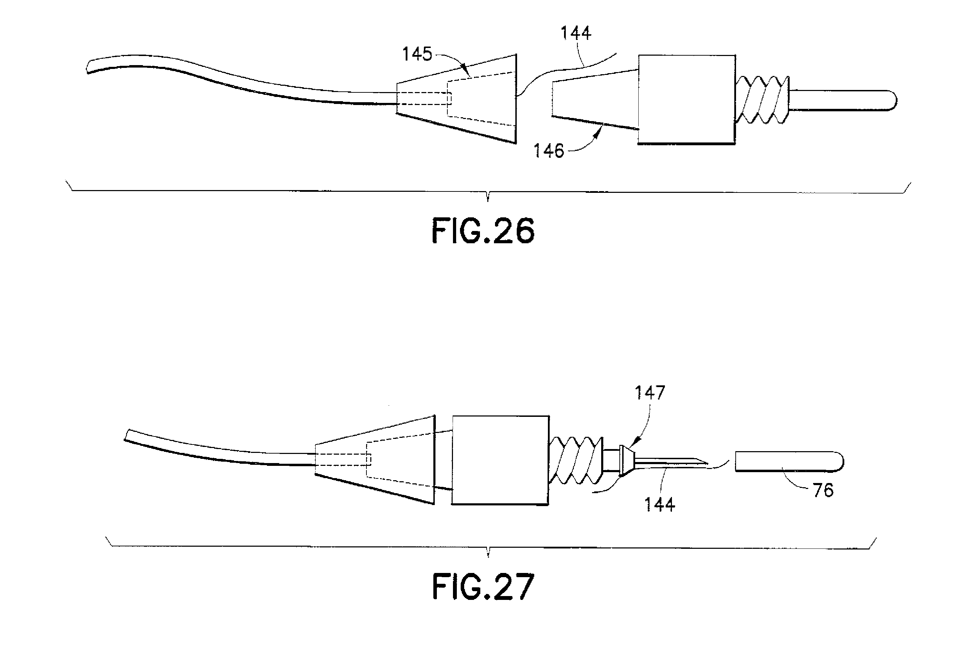

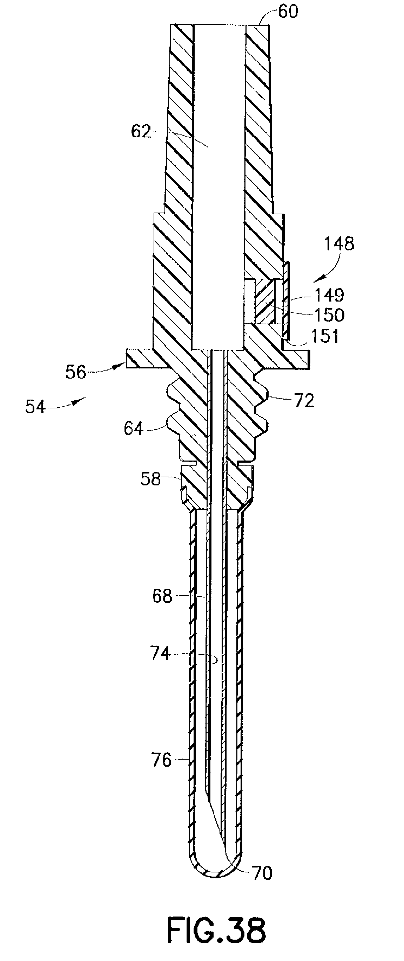

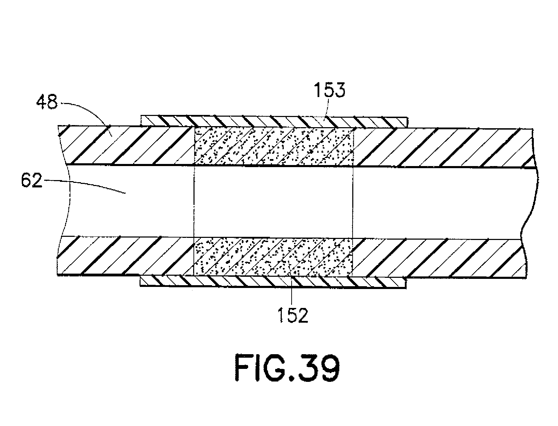

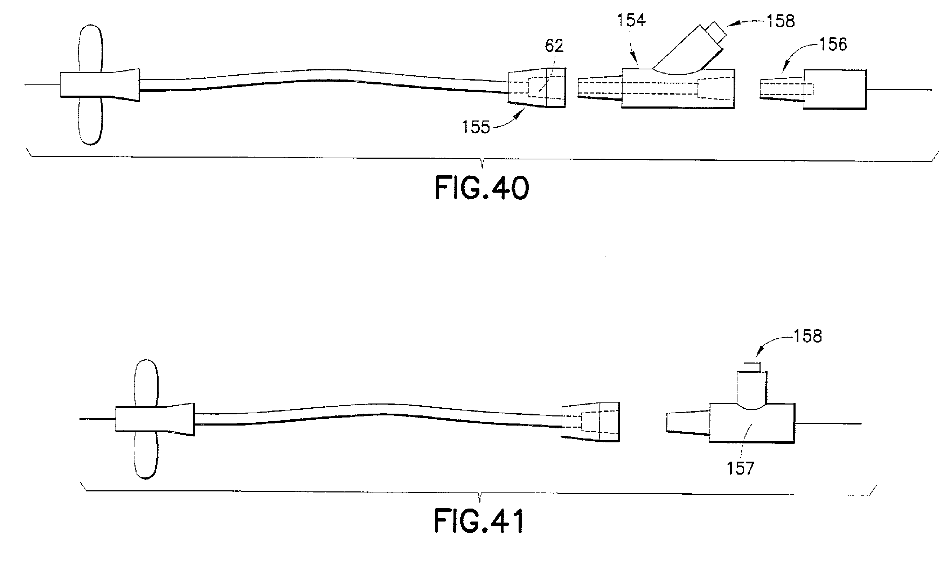

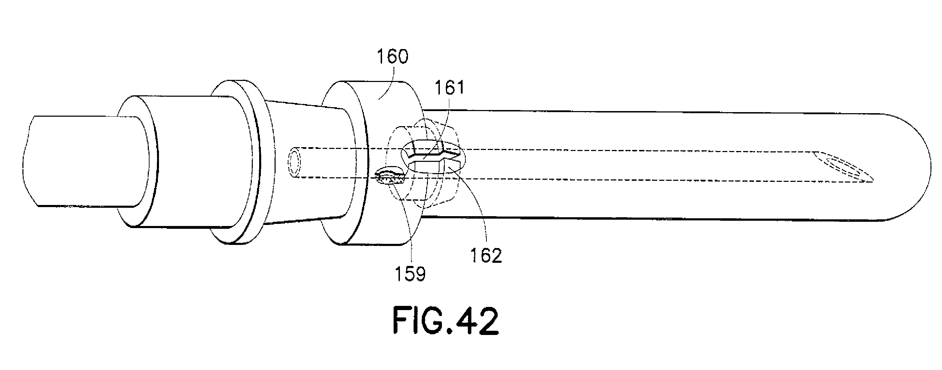

Figs. 3-42 show various embodiments of the invention, including various configurations of venting mechanisms in blood collection tubing sets. In particular, FIGS. 3 to 12 reflect embodiments in which a venting mechanism is located in the female luer 52 portion. FIG. 13 reflects embodiments where the venting mechanism is located between the interface of the female luer **111** and the male luer taper **66.** FIGS. 14-20 reflect embodiments where the venting mechanism is located beyond the proximal end **70** of the non-patient cannula in the non-patient hub **56** FIG. 21 reflects an embodiment where the venting mechanism is remote from the blood collection flow path and that communicates with it through a tortuous path in the non-patient hub. FIG. 22 reflects an embodiment where the venting mechanism is a unified non-patient hub **132.** FIGS. 23 and 24 reflect embodiments where the venting mechanism is a hole in the non-patient cannula. FIG. 25 reflects an embodiment where the venting mechanism is located between male luer wall **125** and the threads **64** of the non-patient hub. FIGS. 26 to 37 reflect embodiments where the venting mechanism is a breathable cord. FIGS. 38 and 39 reflect embodiments where the venting mechanism is a one-way valve. FIGS 40 and 41 reflect embodiments where the venting mechanism is a "Y" or "T" branching in the fluid passage into which the air is displaced FIG. 42 reflects an embodiment of a combination of two venting mechanisms located in the non-patient hub

FIG. 3 shows a venting mechanism that includes an aperture **82** extending radially from the interior of the of the luer **52a** to the exterior. A venting plug **101** is located within at least a portion of the aperture **82.** Aperture 82 thereby provides communication between the fluid passage **62** and the ambient surroundings allowing air to escape but preventing the outflow of blood or other fluids. Venting plug **101** (and like elements described herein) may be formed from any suitable material

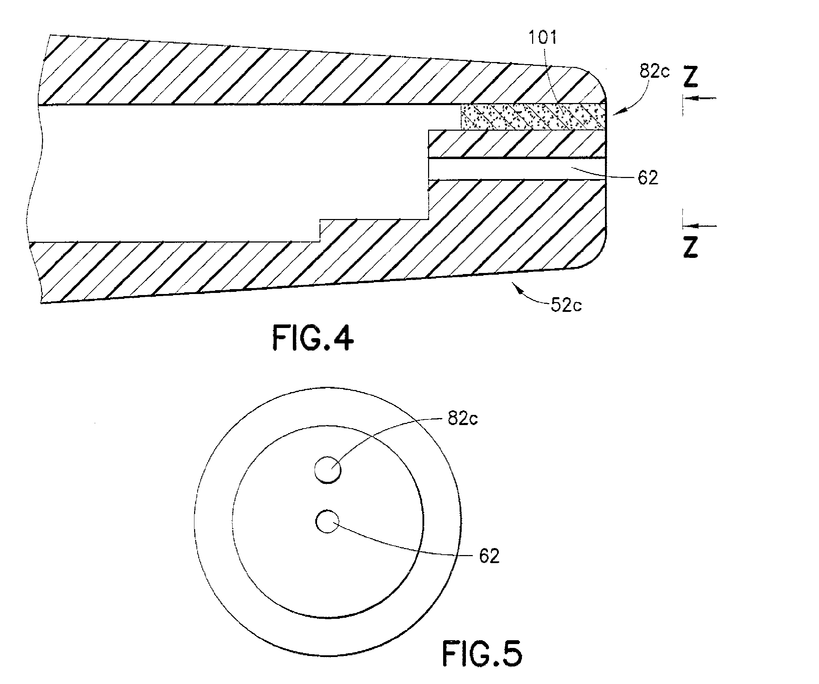

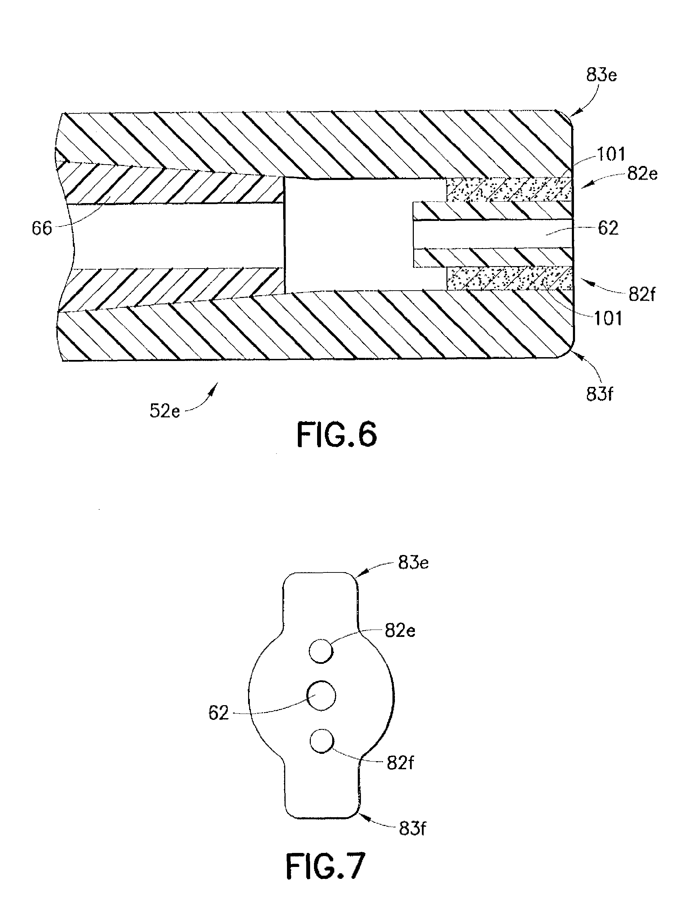

FIG. 4 shows a luer **52e,** having an axially extending aperture **82c** and a venting plug **101** located therein, the aperture running substantially, parallel to the fluid passage **62.** As in the above embodiment, the aperture provides communication between passage **62** of non-patient hub **56** and the ambient surroundings. FIG. 5 shows a side view of Fig 4 from the aspect of Z-Z. An alternative embodiment is shown in FIGs 6 and 7 in which the venting mechanism comprises two axially extending apertures **82e** and **82f** each containing a venting plug **101.** In addition, in this embodiment, the luer **52c** contains two projections **83e** and **83f** in which the apertures are located. In other embodiments, one or more apertures may be located outside the projection. These projections also serve to facilitate the removal of the female luer **52e** off the mating male luer **66**. In alternative embodiments, there may be any number of projections and any vent number of vent apertures. Each projection does not necessarily have a vent aperture

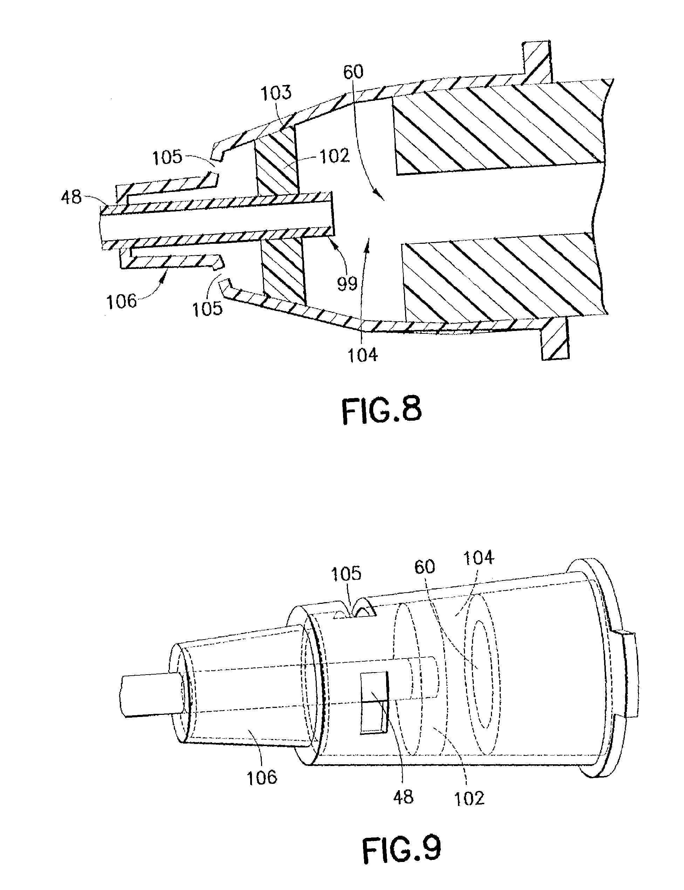

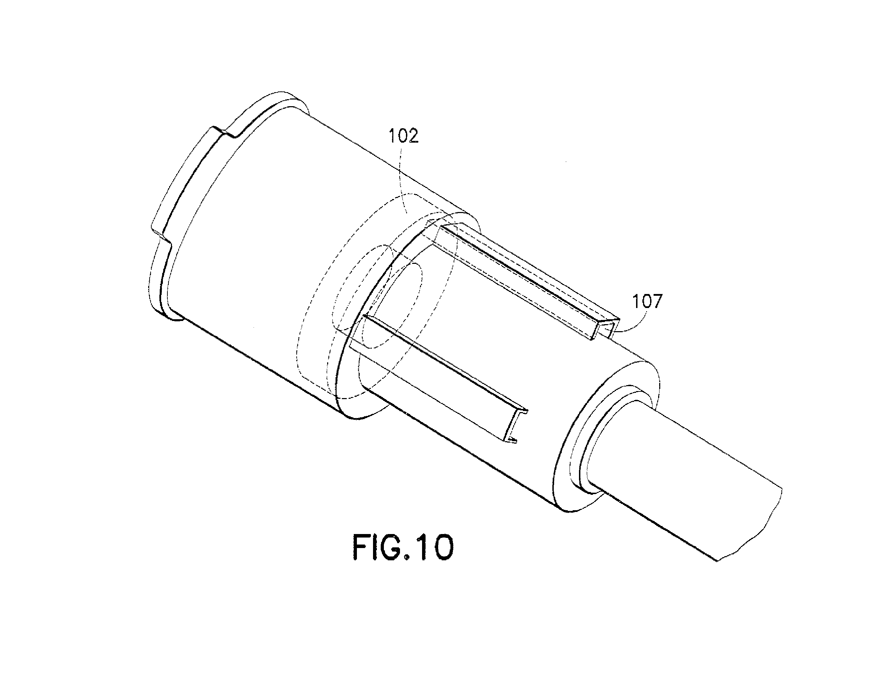

FIGs 8 and 9 show another female luer venting mechanism in which a venting ring **102** is situated around the proximal end **99** of the flexible tube **48.** The venting ring forms an interference fit with the inside female luer taper **103.** A fluid chamber **104** is formed between the interface of the venting ring/ proximal end of the tubing and the non-patient hub distal end **60** when the male luer taper is mated to the female luer, such that air can escape by passing through the venting ring and out of venting apertures **105** to the ambient surroundings. The apertures may be located anywhere in the body of the female luer. In FIGs 8 and 9, the apertures are located in the distal end of the female luer **106.** An alternative embodiment can be send in FIG. 10 in which the venting apertures are longitudinal venting windows **107** which abut the distal side of the venting ring **102**.

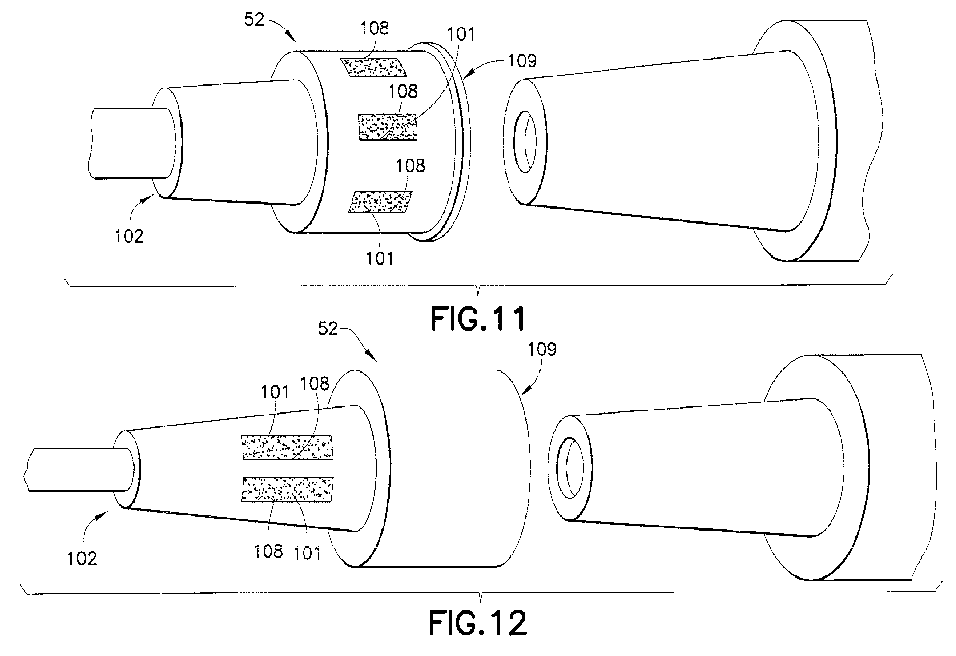

FIG. 11 shows a venting mechanism in which a series of transverse vent apertures **108,** each containing a venting plug **101** are located in the body of the luer **52.** In FIG 11, the apertures are spaced equally around the circumference of the proximal end of the female luer fitting **109.** Similarly FIG. 12 shows a series of transverse vent apertures **108,** each containing a venting plug **101**, that are equally spaced mound the circumference of the distal end of the female luer fitting **52.** In both cases a fluid chamber exists between the male luer taper and the female luer taper surfaces where air can flow out of the fluid passage and out of the vent mechanism.

FIG. 13 shows a venting mechanism that includes a venting plug **101** at a location between the interface of the female luer **111** and the male luer taper **66,** which provides communication between passage **62** of non-patient hub **56** and the ambient surroundings. The venting plug **101** acts as a spacer and forms an interference fit with the inside female luer taper **103.** A fluid chamber **104** is formed between the interface of the female luer taper/proximal end of the tubing **99** and the non-patient hub distal end **60** when the male luer taper is mated to female luer with the venting plug located in-between, such that air can escape by passing through the gap **110** between the male luer and female luer tapered surfaces and the venting plug **101.** Note that in this embodiment, as in the other embodiments herein, the vent material could be a variety of materials or elements. Typically configurations for this embodiment may include, for example, a porous ring formed from a hydrophobic material or which has a hydrophobic surface, a porous ring that becomes seated upon contact with blood using biological phenomena, a ring of swellable material, a textured surface, or a coating of a swellable or similar material

Several embodiments involve a venting mechanism location beyond the proximal end **70** of the non-patient cannula. In the embodiment of Fig. 14, air flows from the fluid passage **62** and out of the non-patient cannula proximal end **70** where it further flows through the space **112** between needle exterior **68** and multiple sample sleeve **76** interior to the location of the vent **113,** which consists of a passage through the non-patient barb **114** then through the venting plug **115** that permits an outflow of air, but prevents an outflow of blood or other fluids to the ambient surroundings.

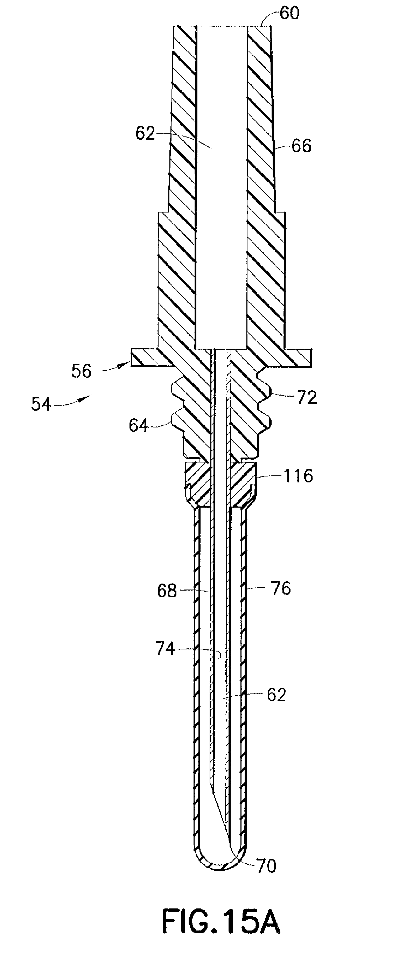

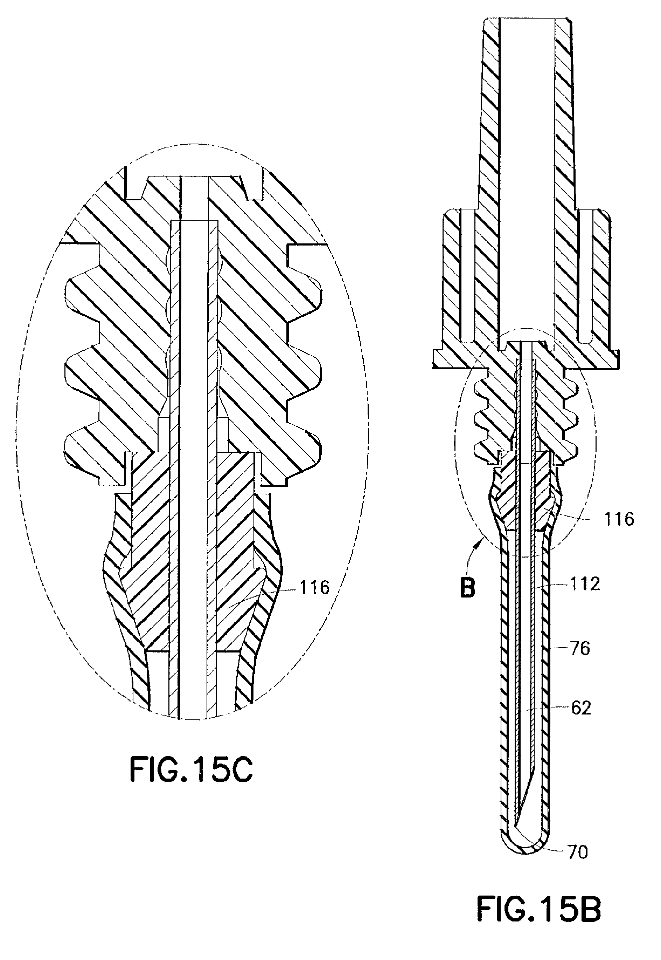

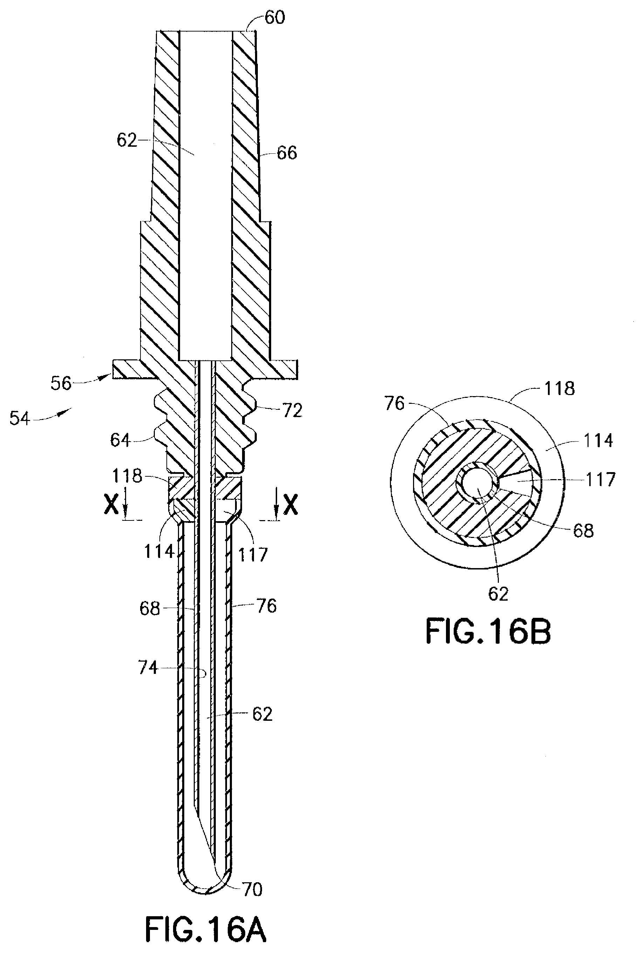

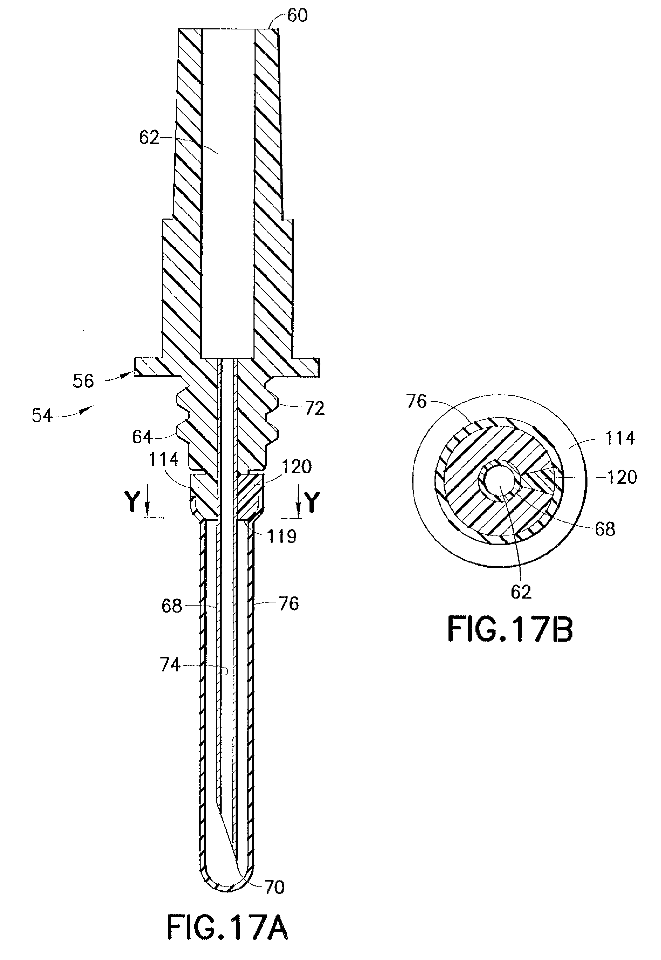

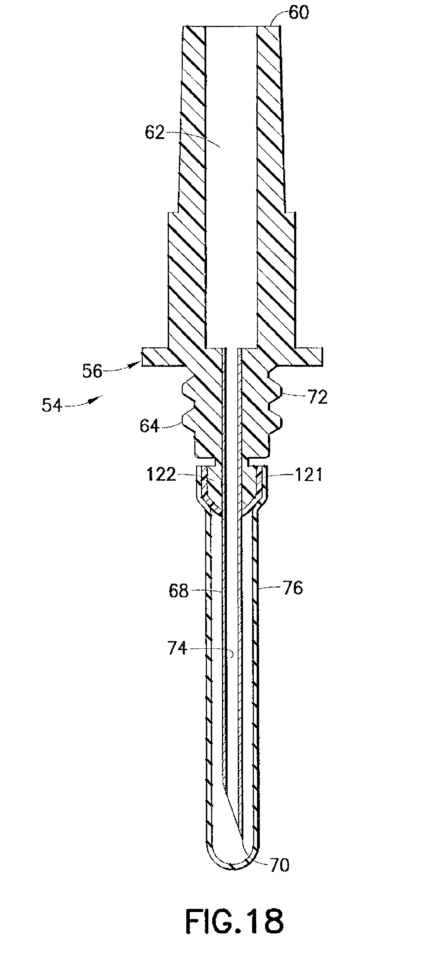

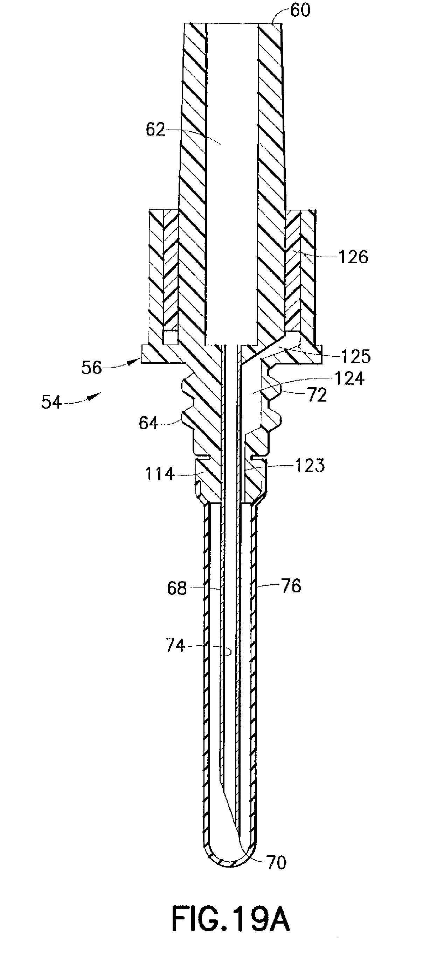

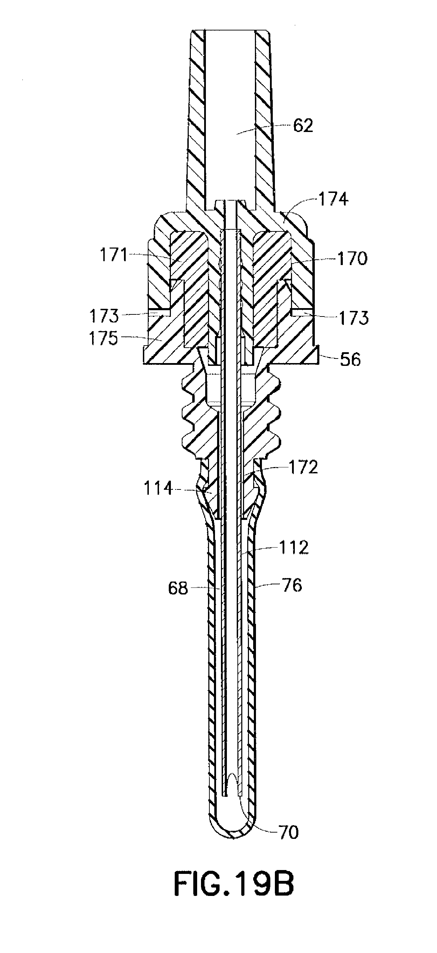

FIGs 15A, 15B, 15C, 16A, 16B, 17A, 17B, 18, 19A and 19B demonstrate more embodiments where venting mechanisms arc located beyond the proximal end **70** of the non-patient cannula. As seen in FIG. 14 air flows from the fluid passage **62** and out of the non-patient cannula proximal end **70** where it further flows through the space **112** between needle exterior **68** and multiple sample sleeve **76** interior to the location of the vent which is situated through or around or part of the non-patient barb. The embodiments in FIGs 15A, 15B and 15C show a venting plug **116** that also functions as the non-patient barb. FIGs 16A and 16B show an embodiment in which a slit **117** in the non-patient barb **114** allows air to escape to a venting disc **118.** FIGs 17A and 17B show an alternative embodiment in which a slit **119** in the non-patient barb **114** contains a venting plug **120** FIG. 18 shows a modified non-patient barb design **122** in which a venting sleeve **121** allows air to escape. FIG. 19A shows a further embodiment in which air escapes through a small channel **123** in the non-patient barb **114** into a thread reservoir **124** then through a channel in the male luer wall **125** into a cylindrical venting plug **126** FIG. 19B shows another embodiment in which the non-patient hub is made from 2 separate parts; the male luer **174** and the non-patient thread assembly **175.** Air escapes through a small channel **172** inbetween the needle exterior **68** and the non-patient barb **114** and then through the male luer wall **56** into a reservoir **170** in the male luer that contains the vent media **171** and out to the surrounding atmosphere through a channel **173** at the interface between the male luer **174** and the non-patient thread assembly **175** of the non-patient hub In each embodiment the vent media (and like elements described herein) permits an outflow of air, but prevents an outflow of blood or other fluids to the ambient surroundings

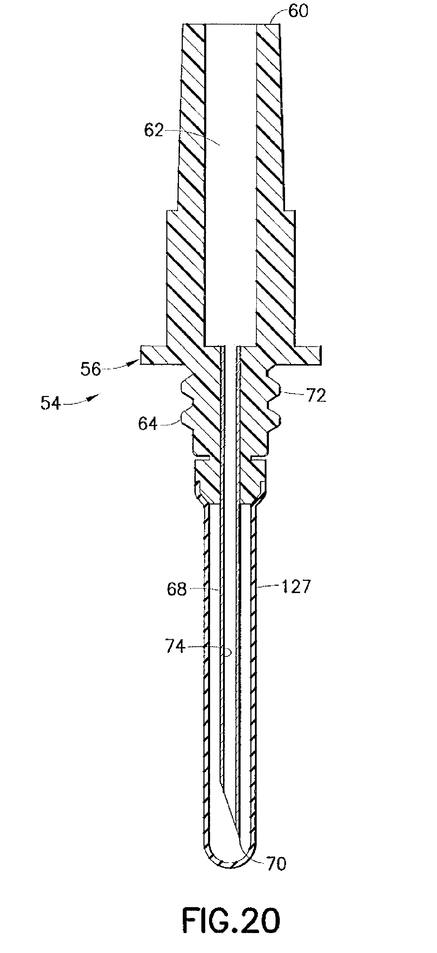

FIG. 20 shows another embodiment, of the venting mechanism location beyond the proximal end **70** of the non-patient cannula. According to this embodiment, air is vented through the material of the multiple sample sleeve **127** itself, which functions as the vent media that prevents a flow of fluid from non-patient cannula **68.** Multiple sample sleeve **127** maintains its normal function of being pierced by pointed proximal end **70** of non-patient cannula **68** in response to forces generated by a stopper on an evacuated collection tube. It is possible to make a multiple sample sleeve 127 that also functions as a vent by forming the sleeve from a porous hydrophobic material, such as those disclosed above

FIG. 21 shows a venting mechanism that is remote from the blood collection flow path and that communicates with it through a tortuous path, thereby preventing contamination of blood in the fluid passage by contact with the vent media. Air flows from the fluid passage **62** within the male luer **128** into a tortuous air pathway **129** that is formed by the male luer **128** and the non-patient cannula hub **130.** Air then passes from the tortuous air pathway **129** to the vent media, which is an annular venting ring **131** and into the ambient surroundings.

FIG. 22 shows a venting mechanism that is a unified non-patient hub **132,** at least a portion of which is made from a porous material. This porous portion of the hub **132** itself thus acts as the vent media The proximal end **99** of the flexible tubing **48** is bonded to the distal end of the unified non-patient hub **133.** The non-patient cannula **68** is bonded to the proximal end of the unified non-patient hub **134**. And because of the nature of the unified non-patient hub **132**, the holder will typically be pro-attached by bonding, rather than by providing threads on the hub **132**. However threads can be provided if desired. Bonding of the tube holder **135** to the unified non-patient hub **134** may be accomplished by solvent, welding, heat, pressure or and other convenient means or combination thereof. Air flows through the fluid passage **62** and vents to the ambient surroundings by passing through the unified non-patient hub **132.** An alternative embodiment incorporates an impermeable spot coat to the inside surface wall **136** of the unified non-patient hub **132** that is part of the fluid passage **62.** This results in air only being able to vent through the hub at the non-patient barb **137** beyond the proximal end **70** of the non-patient cannula.

In the embodiment of FIGs 23 and 24, the venting mechanism is an opening in the non-patient cannula, surrounded by the vent media, which is shown as a vent plug. FIG. 23 shows a hole **138** in the non-patient cannula **68.** Air flows from the fluid passage **62** and out through the hole **138** into a porous venting ring **139** then into the ambient surroundings. In FIG. 24, the hole **140** in the non-patient cannula **68** is situated at the non-patient barb **141,** which also acts as a venting ring. This allows air to escape through the hole **140** that was in the fluid passage between the IV needle assembly and through the non-patient barb **141** for air in a further proximal location down the fluid passage stream after said hole **140**

FIGs 25A and 25B show a venting mechanism that is located between male luer wall **125** and the threads **64** of the non-patient hub. Air flows from the fluid passage **62** into the vent hole **142** and then through the porous C clamp vent plug **143** to the ambient surroundings. As discussed above the vent plug material in this embodiment typically has an elastic property and shape such that spring energy holds the vent material onto the device. In this embodiment the distortion of the C-shaped vent plug shape is required for the vent plug to stretch over the receiving structure on the hub. Once the vent plug is placed over the receiving structure, it is released and fully maintained in place using its own resiliency and in absence of bonding materials such as epoxies, which could be disadvantageously absorbed into the vent plug. The vent mechanism of this embodiment could alternatively involve first compressing the vent material, placing the vent material into the opening, and releasing the vent material to expand into the opening.

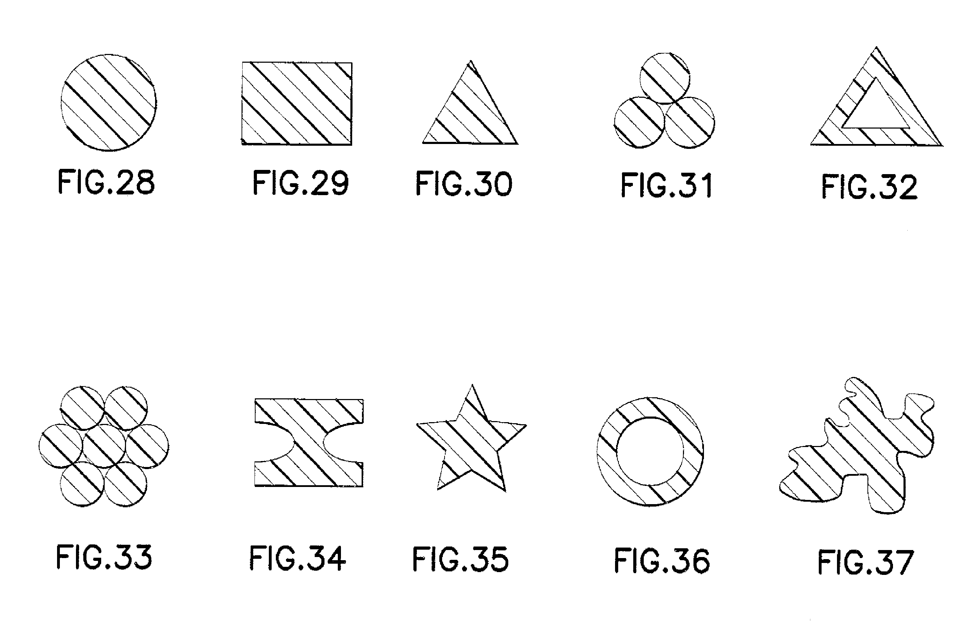

Figs. 26 and 27 show the venting mechanism of a breathable venting cord at two different locations along the fluid passage, though venting using this mechanism may be accomplished by locating the vent media (e.g., placing, coating, or treating) between any one or multiple scaling surfaces along the fluid passage. FIG. 26 shows the cord **144** located between the scaling surfaces of the female **145** and male **146** luer tapers. The presence of the cord in the scaling surface allows air to escape from the fluid passage **62** but prevents leakage of a fluid through either the absorbent nature of the cord material and / or the very small size of the channel created by the cord FIG. 27 shows the cord located between the sealing surfaces of the multiple sample sleeve 76 and non patient hub barb **147** FIGS 28 to 37 show cross-sections of suitable breathable cords. Other shapes, or combinations or such profiles, may also be used. Cords may be extruded or woven, for example and the application of a hydrophobic coating such as wax may be advantageous.

In a further embodiment, the venting mechanism utilizes a one way valve located somewhere along the fluid passage. The valve allows air to escape but shuts closed when vacuum is applied thus, when an evacuated collection tube is applied at the needle tip, the tube draws blood from the fluid passage but not air. Figs. 38 and 39 show examples of a one-way valve. The venting mechanism may be at any location or locations along the fluid passage **62,** but is typically at the proximal end of the non-patient hub **56.** The valve **148** itself may be a thin flap such as plastic film **149** covering the vent, a deformable seal such as a rubber or plastic duckbill valve, a deformable wrap over the vent, or any other means or combination of these. The valve **148** may be proximal or distal with respect to the vent. In the embodiment shown in FIG. 38 the thin plastic film valve **149** is attached to the non-patient hub **56** along one sealed edge of the film **151,** so that on the initial venous puncture, air is pushed out of the fluid passage **62** under venous pressure through the porous vent plug **150** and out from underneath the unsecured edges of the plastic film **149.** However when a vacuum is applied to the fluid passage **62** (via the attachment of a blood collection tube) the thin plastic film valve **149** is pulled tight against the porous vent plug **150** thereby sealing the vent and preventing air from reentering the fluid passage. This embodiment may thus provide a primary or back-up feature to prevent air from re-entering the system after venting occurs. An alternate embodiment is shown in FIG. 39, in which the blood flows from a length of conventional flexible tubing **48** into a venting mechanism that consists of a length of porous tubing **152,** which is loosely wrapped around its outer surface in a length of a non-porous flexible film **153.** This wrap **153** is sufficiently loose to allow air to escape on initial venous puncture such that under a vacuum, it is pulled tight and seals the outer surface of the length of porous tubing **152.**

FIGS. 40 and 41 show a venting mechanism using a "Y" or "T" branching in the fluid passage into which the air is displaced. The branching may be at any location or locations along the fluid passage, but is typically at the proximal end such as at the non-patient hub. FIG. 40 shows a "Y" Branch Vent **154** in the form of a separate component added into the fluid passage **62** such as in between the female **155** and male luer **156** fittings. FIG. 41 shows a "I" Branch Vent **157**, which is an integral part of the non-patient hub, thus reducing the number of components. In each embodiment the vent plug **158** allows the air to escape but prevents leakage of blood. The use of a "Y" or "I" Branch Vent may be accomplished with any convenient terminal shapes at the interfaces of each component. The embodiments exemplified previously are shown with luer tapered fittings but other interface designs such as direct connection to the flexible tubing may also be applied. Although typical applications use a three port "Y" or "I" branch vent the use of a multiple port branch vent system is also possible.

As will be apparent to one skilled in the art, it is possible to combine one or more vent mechanisms in a single device, or put identical vent mechanisms at more than one location in a device Moreover it is possible to use any of a variety of vent plugs in the vent mechanisms of the invention In addition, vent mechanisms herein may be applicable in a variety of devices other than blood collection sets.

For example, FIG. 42 shows a combination of two venting mechanisms Air flows from the fluid passage **62** and out through the hole **159** in the non-patient cannula **68** into a porous venting ring **160** then into the ambient surroundings. A second vent path exists past beyond the proximal end **70** of the non-patient cannula. Air that is proximal to the hole **159** location flows from the fluid passage **62** and out of the non-patient cannula proximal end **70** where it further flows through the space **112** between needle exterior **68** and multiple sample sleeve **76** interior to the slit **161** in the non-patient barb **162** also allows air to escape to the porous venting ring **160.**

Vent media, as used herein, can include, for example, either or a combination of:
- a porous plug formed from a matrix or carrier material, typically hydrophobic, that is coated with, impregnated with, or otherwise contains a hydrophilic material that swells on contact with aqueous or water containing substances. This swellable nature thereby provides the scaling function in the vent upon contact with blood;
- an air vent provided through a matte finish, micro-sized channels, laser drilled holes, tortuous path, or a vent provided between sealing surfaces, e.g., in a cord in which the holes, gaps or channels arc large enough to permit airflow but small enough to prevent, blood leakage;
- a porous plug that becomes sealed upon contact with blood using biological phenomena, eg., by clotting and/or cell agglutination that blocks the vent;
- a superabsorbant material to seal the vent by swelling on contact, with a aqueous fluid; or
- a one-way valve, e.g., a thin flap such as plastic film covering a vent, a deformable seal such as a rubber or plastic duckbill valve, or a deformable wrap over a vent.

Typically, a porous plug is formed from a hydrophobic material, such as high-density polyethylene (IIDPE), which is coated with, impregnated with, of otherwise contains a hydrophilic material such as carboxymethylcellulose (CMC) or a polyacrylate. Alternative hydrophobic materials include but are not limited to polytetrafluoroethylene (PTFE), ultra-high molecular weight polyethylene (IJIIMWPII), Nylon 6, polypropylene (PP), polyvinylidine fluoride (PVDF) or polyethersulfone (PES).

An embodiment of the vent media consists of micro-sized holes formed in an exterior wall. The holes are large enough to permit airflow but small enough to prevent blood leakage. The vent holes may be any number including a single hole although multiple holes arc typical for a more reliable function. The holes may be laser-drilled, meaning that they may be burned through the wall or substrate using one or more laser beams. The substrate may be any convenient material although thin plastic or plastic film is typical. The vent mechanism may include a one-way valve as previously described. The vent mechanism may be located at any convenient space along the fluid passage in the flexible tubing, luer or non-patient hub or in an added component although location at the proximal end is typical to provide flash along the full length of the tubing

A porous plug that becomes sealed upon contact with blood using biological phenomena may use, for example, a porous material such as a sintered plastic, ceramic or metal, or a breathable cord, or by locating the biological agent in small holes or spaces between parts. The vent, may be of any convenient shape. The venting may be at any location or locations along the fluid passage, but is preferably at the proximal end such as at the hub near the collection device. The vent is typically made from contains, is adjacent to, or works in collaboration with, a stimulant that interacts with blood to promote clotting and/or cell agglutination such that the clot and/or clumped cells block ongoing flow of blood through the vent. An example a clotting stimulant is silica or crushed glass, or fiberglass. An example of an agglutinizing agent is lectin. An example of a platelet activator is collagen or thrombin. A neutralizer for anti-coagulant such as protomine sulfate may be included the biological stimulant may be applied using any convenient process including as a powder, a solution, a suspension, a slurry, or any other form. It may be dried or lyophilized.

## Claims

1. A blood collection set comprising;
a first needle assembly having a first hub and a first cannula mounted to said first hub, said first cannula having a lumen extending therethrough,
a length of flexible tubing having opposite first and second ends and a passage extending between said ends, said fust end of said flexible tubing being in communication with said first hub for providing communication between said lumen of said first cannula and said passage through said flexible tubing,
a second hub having a passage extending therethrough and being in fluid communication with said second end of said flexible tubing,
a non-patient cannula having opposite proximal and distal ends and a lumen extending between said ends, said distal end of said non-patient cannula being mounted to said second hub such that said lumen through said non-patient, cannula communicates with said passage through said second hub,
a multiple sample sleeve covering said non-patient cannula and mounted to said second hub.
a venting mechanism providing communication between said passage and ambient surroundings, wherein said venting mechanism permits an outflow of air from said blood collection set to said ambient surroundings through said venting mechanism, and wherein said venting mechanism substantially prevents an outflow of fluid from said blood collection set to said ambient surroundings through said venting mechanism.

2. The blood collection set of Claim 1, further comprising a female luer fitting mounted to said second end of said flexible tubing, such that said venting mechanism extends through said female luer.

3. The blood collection set of Claim 2, wherein said venting mechanism comprises a vent media

4. The blood collection set of Claim 2, wherein said venting mechanism comprises at least one aperture through said female luer, said aperture containing a venting plug

5. The blood collection set of Claim 2, wherein said venting mechanism comprises a venting ring surrounding said second end of said flexible tubing and at least one aperture through said female luer located distally of said venting ring.

6. The blood collection set of Claim 1, further comprising a female luer fitting mounted to said second end of said flexible tubing and a male luer taper formed on said second hub, wherein said female luer fitting is mated with said male luer taper, and wherein said venting mechanism is located between said female luer and said male luer taper.

7. The blood collection set of Claim 6, wherein said venting mechanism comprises a venting plug.

8. The blood collection set of Claim 6, wherein said venting mechanism comprises a porous ring of hydrophobic material or said venting mechanism comprises a ring of porous material with a hydrophobic surface.

9. The blood collection set of Claim 6, wherein said venting mechanism comprises a breathable venting cord

10. The blood collection set of Claim 6, wherein said venting mechanism comprises a textured surface.

11. The blood collection set of Claim 1, wherein said venting mechanism extends through said second hub.

12. The blood collection set of Claim 11, wherein said venting mechanism comprises a vent media.

13. The blood collection set of Claim 11, wherein said venting mechanism comprises a passage through said second hub, said passage containing a venting plug

14. The blood collection set of Claim 13, wherein said venting mechanism further comprises a one-way valve

15. The blood collection set of Claim 1, wherein said venting mechanism is located in said passage beyond said proximal end of said non-patient cannula.

16. The blood collection set of Claim 15, wherein said venting mechanism comprises a vent media

17. The blood collection set of Claim 1, wherein said second hub further comprises a non-patient barb to which said multiple sample sleeve is mounted, and wherein said venting mechanism extends through said non-patient barb.

18. The blood collection set of Claim 17, wherein said venting mechanism comprises a vent media.

19. The blood collection set of Claim 17, wherein said venting mechanism comprises a passage though said non patient bath, said passage containing a venting plug.

20. The blood collection set of Claim 17, wherein said venting mechanism comprises a slit through said non patient barb and a venting disc

21. The blood collection set of Claim 17, wherein said venting mechanism comprises at least one aperture through said non patient barb, said aperture containing a venting plug.

22. The blood collection set of Claim 1, wherein said second hub further comprises a non-patient barb, and wherein said non-patient barb comprises said venting mechanism.

23. The blood collection set of Claim 22, wherein said venting mechanism comprises a vent media.

24. The blood collection set of Claim 22, wherein at least a portion of said non-patient barb is formed from a porous hydrophobic material or said portion of said non-patient barb comprises a porous material with a hydrophobic surface.

25. The blood collection set of Claim 22, wherein said venting mechanism comprises a porous material impregnated with a hydrophilic material.

26. The blood collection set of Claim 1, wherein said second hub further comprises a non-patient barb to which said multiple sample sleeve is mounted, and wherein said venting mechanism is located between said non-patient barb and said multiple sample sleeve.

27. The blood collection set of Claim 26, wherein said venting mechanism comprises a venting sleeve.

28. The blood collection set of Claim 27, wherein said venting sleeve comprises a porous hydrophobic material of said venting sleeve comprises a porous material with a hydrophobic surface.

29. The blood collection set of Claim 26, wherein said venting mechanism comprises a breathable venting cord.

30. The blood collection set of Claim 26, wherein said venting mechanism comprises a textured surface.

31. The blood collection set of Claim 1, wherein said multiple sample sleeve comprises said venting mechanism.

32. The blood collection set of Claim 31, wherein at least a portion of said multiple sample sleeve is formed from a porous hydrophobic material or said portion of said multiple sample sleeve comprises a porous material with a hydrophobic surface.

33. The blood collection set of Claim 1, wherein said venting mechanism comprises a tortuous path providing said communication between said passage and said ambient surroundings.

34. The blood collection set of Claim 33, wherein said second hub further comprises a male luer and a non-patient cannula hub, and wherein said tortuous path is located between said male luer and said non-patient cannula hub.

35. The blood collection set of Claim 1, wherein at least a portion of said second hub is formed from a vent media such that said second hub constitutes said venting mechanism.

36. The blood collection set of Claim 1, wherein at least a portion of said second hub is formed from a porous material such that said second hub constitutes said venting mechanism

37. The blood collection set of Claim 36, further comprising a layer of impermeable spot coat on the inside surfaces of said second hub

38. The blood collection set of Claim 1, wherein said second hub further comprises a male luer and a non-patient thread assembly, which form a reservoir when mated together.

39. the blood collection set of Claim 38, wherein said venting mechanism comprises a first channel through said non patient thread assembly to said reservoir, a second channel from said reservoir to said ambient surroundings, and a venting plug located in said reservoir.

40. The blood collection set of Claim 1, wherein said venting mechanism extends through said non patient cannula.

41. The blood collection set of Claim 40, wherein said venting mechanism comprises a vent media.

42. The blood collection set of Claim 40, wherein said venting mechanism comprises a hole through said non patient cannula and a porous venting ring.

43. The blood collection set of Claim 40, wherein said venting mechanism comprises a hole through said non patient cannula and a resilient porous vent plug, wherein said resilience of said vent plug secures said vent plug in location around said hole

44. The blood collection set of Claim 1, wherein said venting mechanism extends through said flexible tubing.

45. The blood collection set of Claim 44, wherein said venting mechanism comprises a vent media.

46. The blood collection set of Claim 44, wherein said venting mechanism comprises a length of porous tubing and a one-way valve wrapped around said length of porous tubing.

47. The blood colleclion set of Claim 1, further comprising a branch vent in communication with said passage.

48. The blood collection set of Claim I, wherein said second hub further comprises a Y branch vent or a T branch vent in communication with said passage.

49. The blood collection set of Claim 1, wherein said venting mechanism comprises a vent media.

50. The blood collection set of Claim 1, wherein said venting mechanism comprises a porous hydrophobic material or said venting mechanism comprises a porous material with a hydrophobic surface.

51. The blood collection set of Claim 50, wherein said hydrophobic material is selected from the group consisting of glass fiber, high-density polyethylene (HDPE), polytetrafluoroethylene (PTFE), ultra-high molecular weight polyethylene (UHMWPE), Nylon 6, polypropylene (PP), polyvinylidine fluoride (PVDF) and polyethersulfone (PES)

52. The blood collection set of Claim 1, wherein said venting mechanism comprises a hydrophilic material.

53. The blood collection set of Claim 52, wherein said hydrophilic material is carboxymethylcellulose or a polyactylate.

54. The blood collection set of Claim 1, wherein said venting mechanism comprises;
a porous plug; and
a material that swells on contact with aqueous substances

55. The blood collection set of Claim 1, wherein said venting mechanism comprises an air vent, wherein said air vent comprises at least one element selected from the group consisting of a matte finish, micro-sized channels, laser drilled holes, and a tortuous path.

56. The blood collection set of Claim 1, wherein said venting mechanism comprises a breathable venting cord.

57. The blood collection set of Claim 1, wherein said venting mechanism comprises a one way valve.

58. The blood collection set of Claim 1, wherein said venting mechanism comprises a porous plug and a biologically active agent.

59. The blood collection set of Claim 1, wherein said venting mechanism comprises a superabsorbant material.

60. The blood collection set of Claim 1, further comprising a shield for selectively covering said first cannula.

61. The blood collection set of Claim 1, further comprising a second venting mechanism

62. The blood collection set of Claim 1, further comprising at least one wing attached to said first needle assembly.
